# EUROPEAN PATENT SPECIFICATION

(11) **EP 3 298 028 B1**
(45) Date of publication and mention of the grant of the patent: **06.11.2019**
(21) Application number: 16722375.9
(22) Date of filing: 31.03.2016
(51) Int. Cl.: C07K 14/47, G01N 33/68

(54) **METHOD FOR DETECTING AND MEASURING ENDOGENOUS COMPLEXES AS A NEW TUMOUR MARKER**
VERFAHREN ZUR ERKENNUNG UND MESSUNG VON ENDOGENEN KOMPLEXEN ALS EIN NEUER TUMORMARKER
PROCÉDÉ DE DÉTECTION ET DE MESURE DE COMPLEXES ENDOGÈNES EN TANT QUE NOUVEAU MARQUEUR TUMORAL

(30) Priority: 20.05.2015 IT UB20150674
(43) Date of publication of application: 28.03.2018
(73) Proprietor: Finotti, Paola, 35028 Padova (IT)
(72) Inventor: FINOTTI, Paola, 35028 Padova (IT); TRAMENTOZZI, Elisa, 35037 Teolo (PD) (IT)
(74) Representative: Vinci, Marcello
(86) International application number: PCT/IB2016/051839
(87) International publication number: WO 2016/185296

(56) References cited:
- WO-A1-2009/013097
- -: "Diagnostic and prognostic biomarkers in both type 1 diabetes and in solid tumors", www.bolsatecnologia.pt , 23 April 2015 (2015-04-23), XP055239938, Retrieved from the Internet: URL:https://web.archive.org/web/2015042309 2119/http://www.bolsatecnologia.pt/Profile View.action?id=29296 [retrieved on 2016-01-11]
- PAGETTA ET AL: "Characterization of immune complexes of idiotypic catalytic and anti-idiotypic inhibitory antibodies in plasma of type 1 diabetic subjects", MOLECULAR IMMUNOLOGY, PERGAMON, GB, vol. 44, no. 11, 16 March 2007 (2007-03-16), pages 2870-2883, XP005938371, ISSN: 0161-5890, DOI: 10.1016/J.MOLIMM.2007.01.012
- TRAMENTOZZI E ET AL: "Stable complexes formed by Grp94 with human IgG promoting angiogenic differentiation of HUVECs by a cytokine-like mechanism", MOLECULAR IMMUNOLOGY, PERGAMON, GB, vol. 45, no. 13, 1 August 2008 (2008-08-01), pages 3639-3648, XP002501489, ISSN: 0161-5890, DOI: 10.1016/J.MOLIMM.2008.04.020 [retrieved on 2008-06-12]
- ANDREA PAGETTA ET AL: "Structural Insights into Complexes of Glucose-Regulated Protein94 (Grp94) with Human Immunoglobulin G. Relevance for Grp94-IgG Complexes that Form In Vivo in Pathological Conditions", PLOS ONE, vol. 9, no. 1, 1 January 2014 (2014-01-01) , page e86198, XP055239922, DOI: 10.1371/journal.pone.0086198

## Description

### FIELD OF THE INVENTION

The invention pertains to a new method of immune-detection for the measurement in human blood of complexes of Glucose-regulated protein94 (Grp94) with Immunoglobulin G (IgG) as diagnostic and prognostic marker of tumor.

### STATE OF THE ART

In the strenuous, continuous effort to identify the molecular mechanisms underlying the development of tumors and to find the most effective anti-tumor therapies, a particular attention has recently been focused on a group of intra-cellular proteins, "Glucose-regulated proteins" (Grps) belonging to the family of the "Heat Shock Proteins" (HSPs). As suggested by the name, the expression of these proteins is induced by diverse stress stimuli, both intra- and extra-cellular, such as hypoglycemia, hypoxia, viral infections, cell proliferation, and the genetic defects that lead to accumulation of structurally altered proteins (Lee A.S., Nat. Rev. Cancer 2014; 14: 263-276). At variance with other HSPs that are prevalently confined to the cytoplasm and/or the nucleus, Grps are localized in the endoplasmic reticulum (ER), the site specifically deputed to defend the cell against stress stimuli. The most important Grp, not only for being the most abundant in the ER, is Grp94 that, similarly to other Grps, is constitutively expressed in any cell but is actively transcribed under stress conditions (Eletto D., et al., Sem. Cell & Dev. Biol. 2010; 21: 479-485). Besides displaying the activity shared by other HSPs, i.e., to confer on nascent proteins the correct conformation and to favor degradation of altered proteins, Grp94 also possesses the unique property to modulate the immune system (Randow F. and Seed B., Nat. Cell Biol. 2001; 3: 891-896*;* Wu S., et al., J. Biol. Chem. 2012; 287: 6735-6742). This specific function is due to the capacity of Grp94 to load cellular antigenic peptides that are to be exposed to the cell surface and presented to immune-competent cells in order to induce an antigen-specific immune response (Chhanda B., et al., Int. Immunol. 2006; 18: 1147-57)*.*

A lot of papers have so far been published that also stress the role of Grp94 in the pathogenesis of tumors, both solid and hematological (Feldweg A.M. and Srivastava P., Int. J. Cancer 1995; 63: 310-314*;* Boelens J., et al., Leukemia Res. 2013; 37: 3-8*).* The intense proliferation characterizing the tumor cells not only induces the over-expression of Grp94 but also causes the translocation of Grp94 from the ER to the cell surface. Indeed, by binding antigenic peptides Grp94 loses a particular C-terminal amino acidic sequence that is responsible for the retention of the protein in the ER (Capitani M., Sallese M., FEBS Lett. 2009; 583: 3863-3871*).* With the loss of the physiological intra-cellular location and the appearance on the cell surface (and also in the extra-cellular space), Grp94 acquires new, different functions. It behaves as a potent cytokine with inflammatory and immune effects that influence the cell growth and invasiveness, and modulate the cell signaling systems (Lee A.S., Nat. Rev. Cancer 2014; 14: 263-276*;* Tramentozzi E., et al., Mol. Immunol. 2008; 45: 3639-3648*).*

The role of Grp94 in the development and progression of tumors has been demonstrated in a large number of investigations (Zheng H-C., et al., Human Pathol. 2008; 39:1042-1049*;* Takahashi H., et al., Histol. Histopathol. 2011; 26: 663-671*;* Dejeans N., et al., Free Rad. Biol. & Med. 2012; 52: 993-1002). The fact that Grp94 is recognized as one of the most diffuse antigens expressed in tumors of various types and at different stages of evolution, makes this HSP be not only a useful tool for the diagnosis and prognosis of disease but also a potential therapeutic target (Schöntal A.H., et al., Biochem. Pharmacol. 2013; 85: 653-666*;* Slotta-Huspenina J., et al., PloSone 2012; doi: 10.1371/journal.pone.0041420). Indeed, the exposure of Grp94 on the surface of cancer but not of healthy cells, offers the opportunity to achieve a cancer-specific therapy without damaging healthy tissues/organs. In this respect, several research groups have realized various strategic approaches to target the protein with molecules that, despite their distinct chemical properties, could all block the Grp94 function to sustain the growth and spreading of the tumor (Patel P.D., et al., Nat. Chem. Biol. 2013; 9: 677-682*;* Lee A.S., Nat. Rev. Cancer 2014; 14: 263-276*;* Rachidi S., et al., J. Hepatol. 2014; 62: 879-888). A different therapeutic approach predicts that Grp94 can instead be employed as a tool to cure tumors and, following this indication, numerous Grp94-based vaccines have been developed that exploit the capacity of Grp94 to activate the immune function (Lee K.P., et al., Hematol. Oncol. Clin. N. Am. 2006; 20: 637-659*;* Schreiber T.M., et al., Cancer Res. 2009; 69:2026-2033*;* Wang X-Y, et al., J. Immunol. 2010; 184: 6309-6319*;* Wang S., et al., Vaccine 2011; 29: 6342-6351*).* The aim of the vaccine-based therapeutic approach is therefore to induce in the patient an effective immune response against the tumor by directing cytotoxic lymphocytes against the cells presenting the antigen (Grp94) on their surface.

From this premise it follows that in either one of the two therapeutic approaches, the pharmacological one, with molecules blocking the tumor-supporting functions of Grp94, or the immune stimulant approach, with a Grp94-based vaccine, the fundamental prerequisite is that Grp94 is easily detected as tumor antigen. Thus, it is rather strange that no diagnostic tool has so far been developed to measure Grp94 in blood and other biological fluids. Indeed, the numerous ELISA (*Enzyme Linked Immunosorbent assay*) kits presently available on the market for the measurement of Grp94 are conceived and delivered for the research purpose only. However, in order to consider Grp94 a tumor biomarker of diagnostic value and also of utility for predicting the efficacy of anti-tumor therapy, it is necessary that Grp94 can be measured as a circulating antigen.

### BACKGROUND AND AIM OF THE INVENTION

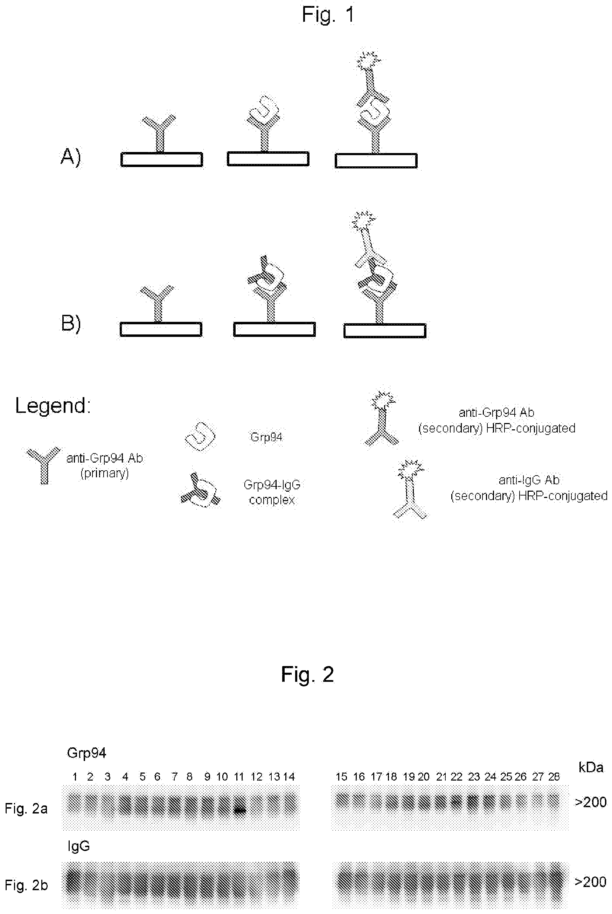

The experiments that allowed us to conceive and set up an immune-detection method useful for measuring Grp94 in biological fluids to diagnostic purpose, originated from previous works performed on plasma of type 1 diabetic subjects, with the demonstration that Grp94 was present in plasma in high concentrations and always in complexes with IgG (Pagetta A., et al., Diabetologia 2003; 46: 996-1006*;* Pagetta A., et al. Mol. Immunol. 2007; 44: 2870-2883*).* The presence of Grp94 in the blood of diabetic subjects had a clear pathological meaning, being the proof of ongoing cell damage induced by the autoimmune process responsible for the onset of type 1 diabetes (Pagetta A., et al. Mol. Immunol. 2007; 44: 2870-2883). The finding that Grp94 was present in complexes with IgG was attributed to the property of extra-cellular Grp94 to behave as potent immunogen, with the capacity to stimulate the production of specific antibodies, a reason for which the complexes were initially identified as immune in nature (Tramentozzi E., et al., J. Cell Mol. Med. 2009; 13: 1336-47)*.* However, in following *in vitro* experiments it was ascertained that Grp94 was also able to bind IgG through a non-immune binding, that turned out to be particularly stable and resistant to several denaturing conditions (Tramentozzi E., et al., Mol. Immunol. 2008; 45: 3639-3648*;* Pagetta A., et al., PloSone 2014; doi: 10.1371/journal.pone. 0086198*).* It was thus hypothesized that the same non-immune binding observed *in vitro* could also take place between Grp94 and IgG *in vivo* whenever Grp94 was liberated outside the cell, and that circulating complexes could therefore be prevalently non-immune in nature. A proteomic investigation conducted on the blood of diabetic subjects actually confirmed this hypothesis, showing that Grp94 present in the circulation was only that irreversibly linked to IgG through a non-immune linkage (Roveri A., el al., J. Diabetes Res. 2015; Article ID 815839*).* It is well known that in an immune complex the antigen-antibody linkage is reversible; thus, although it is reasonable to suppose that Grp94 can form immune complexes with IgG from which it can easily detach, the accumulated experimental evidence indicates that Grp94 cannot remain free for long, being rapidly and stably involved in a non-immune binding with IgG. These premises help understand why it was so difficult to measure circulating Grp94 using the ELISA method that is based on the principle to detect and measure a biological target through the antigenic recognition by specific antibodies. Indeed, the ELISA kits presently available on the market can detect and measure only the single protein (Fig. 1, A) like that obtained after purification from different biological sources, but they cannot give a reliable measurement of Grp94 as it actually is *in vivo,* i.e., in complexes with IgG.

In document WO2009/013097 (*Finotti P., Brevetto Nazionale 2007, N°*PD2007A000252) of the same applicant dealing with the method followed to obtain *in vitro* the complexes of Grp94 with IgG, the potential therapeutic use of the complexes formed *in vitro* and the possible exploitation of these complexes to measure circulating antibodies against the complexes were claimed. In that patent, however, the *in vitro* formed complexes were predicted to be used exclusively as the capture agent in the ELISA method, but in no part of the patent the possibility was ever claimed to measure endogenous complexes as a marker of tumor and index of the extent of extra-cellular liberation of Grp94.

In a following document "*Diagnostic and prognostic biomarkers in both type 1 diabetes and in solid tumors*" (www.bolsatecnologia.pt; TOIT20140423001), the use was claimed of an ELISA kit to measure endogenous Grp94-IgG complexes and antibodies against these complexes. However, the method ELISA described in that document implied the use of the Grp94-IgG complexes formed *in vitro* (matter of the National Patent 2007) and thus exploited a product and used a procedure of measurement different from those proposed in the present patent.

Overall, both the methods described in the above documents gave unsatisfactory results as far as the measurement of endogenous Grp94-IgG complexes is concerned, thus, a series of new experiments was undertaken to develop a sandwich ELISA with anti-Gpr94 antibodies as coating agent that is matter of the present application. This new method does not employ any more the *in vitro* formed Grp94-IgG complexes to measure the immune reaction, and has allowed us to obtain a reliable quantitative measurement of the protein under exam.

### DETAILED DESCRIPTION OF THE INVENTION

The immunoassay for the determination of Grp94-IgG complexes intended for diagnostic and prognostic purpose in the tumor pathology, is a sandwich ELISA in which the measurement of the protein under exam is performed with the aid of two types of antibodies: the primary (capture) antibody, preferentially bound to the plate wells and reacting with one or more antigenic determinants of one of the two proteins forming the Grp94-IgG complex in the biological sample, and the secondary (detection) antibody directed against one or more antigenic determinants of the other protein forming the complex. The said secondary antibody is preferentially, but not exclusively, conjugated with an enzyme that makes the immune reaction be visible through the conversion of a specific substrate into a colored product detected by reading the absorbance at a specific wavelength. The presence of the complex can thus be revealed by the detection system of the antibody linked to the second protein of the complex only if the second protein is stably linked to the first protein of the complex that is bound to the primary antibody (Fig. 1, B).

The procedure implies the use of 96-well plates with high absorbance capacity for antibodies. The sequence of steps to follow is listed hereinafter.
**1)** Coating step. The plate is coated with primary (coating) anti-Grp94 polyclonal antibodies that recognize human Grp94. The antibodies are diluted in a buffer solution consisting, preferentially, but not necessarily, of 0.05 M carbonate/bicarbonate (pH 9.6) to obtain a final concentration of 2-4 µg/ml (working solution), 50 µl of the working solution are put in each well and the plate is then sealed and left in incubation preferentially overnight at +4 °C.
**2)** After incubation, the coating solution is discarded and wells filled with 300 µl of the washing solution A (PBS, phosphate buffered saline or any other buffer solution). The washing liquid is discarded in a waste container and the wells sharply struck on an absorbent paper to remove residual buffer. This step is repeated twice.
**3)** Wells are filled with 300 µl of a blocking solution (with bovine albumin from 2% to 4% in PBS or with another type of albumin or blocking substance at 2% to 4% in a buffer solution), leaving the plate in incubation with coverage, preferentially, but not necessarily, at room temperature for 1 h.
**4)** The plate is washed as described in step 2) and the washing is repeated three times.
**5)** 100 µl of the biological sample are added, at least in duplicate, in predesigned wells (see below for detailed description of the biological sample). The biological sample is preferentially, but not exclusively, made with plasma, serum or other biological fluid, and can be used at scalar dilutions preferentially in a buffered solution with PBS and Tween at concentrations from 0.05% to 0.3% (preferentially at 0.1%), with the addition of one or more blocking substances at concentrations from 0.5% to 10%.
6) The plate is left covered in incubation, preferentially at room temperature for 1-2 h.
**7)** The plate is washed as described in step 2) for three times with 300 µl of the washing solution B containing PBS (or other buffer solution) and Tween at concentrations from 0.03 to 0.15% (preferentially at 0.05%).
**8)** Secondary (detection) antibodies are added that are anti-human polyclonal antibodies obtained from different animal species (donkey or another animal) and are preferentially conjugated with horseradish peroxidase (HRP) or associated with another system of detection and amplification of the signal related to the immune reaction. Secondary antibodies can be formed with whole IgG or fragments of IgG (Fab₂) conjugated either directly or indirectly (through amplification systems) with the detection system. Secondary antibodies are diluted with the washing solution B (dilutions from 1:5,000 to 1: 20,000), following the manufacturer's instructions. 100 µl of this solution are then added to each well. The plate is covered and kept in the dark preferentially for 30 min.
**9)** The plate is washed as described in step 7).
**10)** Preparation of the substrate solution to detect the immune reaction. When secondary antibodies are conjugated with HRP, an equal volume of the chromogen substrate 3,3',5,5'-tetramethylbenzidine (TMB) and of hydrogen peroxide is mixed in the citric acid buffered solution (pH 3.3), and 100 µl of the substrate solution are added in each well. As an alternative to the TMB solution, other substrates and substrate solutions ready for use can be employed as well. The plate is covered and left in incubation in the dark on a shaker for 5-20 min.
**11)** 100 µl of the blocking solution (stop solution) are added in each well to block the reaction. The stop solution is preferentially, not necessarily, made with 0.3 M sulfuric acid.
12) The intensity of the colorimetric reaction, taken as index of the immune reaction, is evaluated by reading the absorbance at a specific wavelength on an ELISA plate reader. Reading is made immediately after the development of the colorimetric reaction, in any case not after 30 min from the development of the reaction.

### EXPERIMENTS AND RESULTS

To test the validity of the proposed methodological approach, experiments were conducted on a sample of patients admitted to the division of General Surgery of the Civil Hospital of Padua for surgical resection of primary tumors of the gastro-enteric tract (oesophagus, stomach, colon, sigma/rectum). Criteria for the enrollment of patients were that patients did not receive any prior anti-tumor treatment and that tumor was diagnosed for the first time. The study protocol was submitted to patients for their informed written approval. The protocol also included the drawing of a blood sample (15 ml) the day before the surgical intervention. A similar blood sample was also obtained from a sample of healthy control subjects recruited among those attending the Department of Pharmaceutical and Pharmacological Sciences of the University of Padua (students and regular workers) after the subjects gave their informed written consent. The study protocol was approved by the Ethics Committee of the Regione Veneto.

The aim of the study was to see whether in the blood of patients Grp94 was detected in the form of complexes with IgG, thus validating the assumption that Grp94-IgG complexes could be a marker of tumor irrespective of the tumor type.

Plasma was obtained by centrifuging blood for 10 min at 3,500 rpm and after the protein concentration was determined on each plasma sample, plasma was stored at -80 °C in aliquots ready to use. On each plasma sample, Western blot analysis was preliminarily performed to test the presence of Grp94; the analysis also served as reference for comparisons to be made with the measurements of Grp94 with the ELISA method. Although Western blotting is a sensitive and reliable method to detect a protein, it nevertheless gives only a qualitative measurement of the protein and cannot be used for screenings on a large scale given the laborious and expensive procedure, a reason for which its application is limited to the laboratory research only.

### Preparation of the biological sample

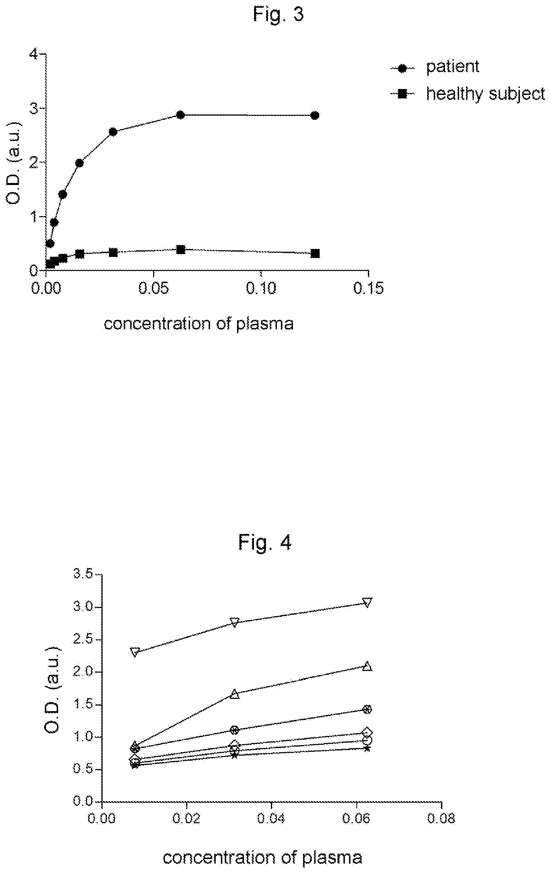

The ELISA method used for the quantitative measurement of Grp94-IgG complexes was tested on plasma samples of both cancer patients and healthy control subjects. Each plasma sample was previously submitted to Western blot analysis to verify the presence of Grp94. Preliminary experiments were set up in which the plasma of some patients and of healthy control subjects was analyzed at scalar dilutions, in duplicate for each dilution, following the procedure described above (steps in "Detailed Description of the Invention"). Experiments were finalized to assess the pattern with which the immune reaction increased in intensity as a function of the plasma concentration and to establish the dilutions with which the reaction was linear. To this aim, a patient of reference was first chosen who developed a particularly intense reaction for Grp94 in the Western blot analysis, thus serving as positive control (Fig. 2, patient #11). The plasma of this patient was tested in parallel with that of a healthy control subject at scalar dilutions (from 1:516 to 1:16) (Fig.3). It can be observed that only in the patient the absorbance (index of the intensity of the immune reaction for Grp94-IgG complexes) rose progressively and significantly by increasing the plasma concentration (expressed as decreasing values of dilution) up to reach the plateau at the lowest dilution of 1:16.

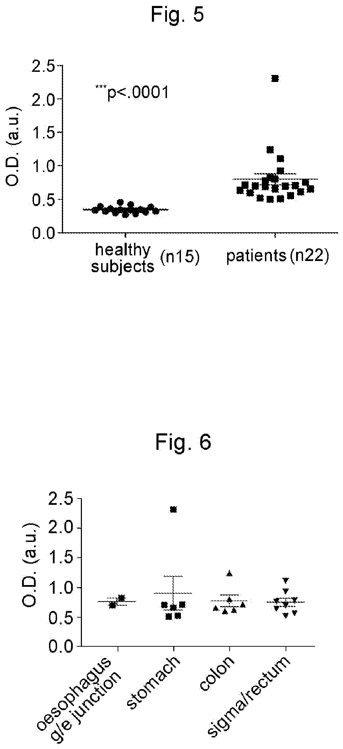

After this preliminary experiment, the analysis was performed on additional 5 patients whose plasma was used at the dilutions of 1:128, 1:32 and 1:16 corresponding to those that in the absorbance curve obtained with the plasma of the reference patient yielded a linear increase of the reaction. It was thus observed that in any patient, despite inter-individual variability, the intensity of the immune reaction increased linearly with the increase of the plasma concentration (Fig. 4). We then extended the analysis to other patients and control subjects to determine the specificity and sensitivity of the biological marker. To this aim, the ELISA test was conducted on plasma at the single dilution of 1:128 considered the most appropriate to include both highest and lowest measurements of the immune response expected to occur in the group of patients. The statistical analysis of data showed that the mean (± SEM) value of absorbance (in arbitrary units) in healthy control subjects was 0.34 (± 0.013) (*range*: 0.27-0.45) and in patients 0.796 (± 0.081) (*range*: 0.51-2.31); the difference between the means was statistically highly significant (p<0.0001) (Fig. 5). An elevated dispersion of values was observed in patients due to expected different concentrations of complexes in plasma, whereas control subjects had particularly low and homogeneous values of absorbance (Fig. 5). These results allowed us to fix the upper limit of absorbance (cut-off) in healthy control subjects to 0.45 units, a value that sharply separated healthy from diseased subjects. The sensitivity and specificity of the assay calculated in our sample was thus 100%: all patients had values of absorbance higher than 0.5 units (no false negative) and no one of healthy control subjects had values higher than 0.45 (no false positive).

Since our patients had primary solid tumors originating from different anatomical sites in the gastro-enteric tract, and were thus non homogeneous for the type of tumor, it was of interest to analyze the measurements made with the ELISA test after patients were grouped for site of tumor (Fig. 6). The dispersion of values of the tumor marker noted when patients were analyzed together (Fig. 5) might have been attributable to differences in the tumor type. It was observed that there was not any significant difference among the means (and SEM) of values of patients grouped by tumor type (Fig. 6). Of note was the finding that the patient with the highest value of absorbance (i.e., the highest concentration of complexes in plasma) also had an exceptionally elevated value of circulating endothelial cancer cells (data not shown), to testify the extensive metastatic diffusion of the tumor. The same patient died two months after he underwent tumor excision and blood examination.

On the whole, results proved that the marker was a valuable diagnostic tool for tumors of the gastro-enteric tract irrespective of the tumor type. The marker can therefore be profitably employed to diagnostic and prognostic purposes of solid tumors of different histological type and anatomical site. As stated in the introductory part, the necessary condition that makes Grp94-IgG complexes fulfill the requisite of a tumor marker is that Grp94-IgG complexes can be detected as circulating antigen; such a condition is expected to be even better satisfied for hematological tumors, a reason for which Grp94-IgG complexes might assume the role of general tumor biomarker.

That the new ELISA method for measuring Grp94-IgG complexes in plasma satisfies the criteria of reliability and effectiveness is proved by the fact that measurements obtained with the new ELISA method fit results of Western blotting made on the same plasma samples (Fig. 2). Although the two methods cannot be compared since the Western blot analysis does not permit to make any quantitative inference about the entity of the immune reaction, it is nevertheless worth noting that patients with the highest values of absorbance were also those who in Western blot analysis displayed a more intense immune reaction for Grp94 (for example, patient # 11).

In our investigation conducted on a relatively small number of patients, measurements of Grp94-IgG complexes are expressed as arbitrary units of absorbance, taken as index of the intensity of the immune reaction. However, the conversion into concentration units (ng/ml) of the complex can be easily obtained by reading the absorbance units of unknown plasma samples on the calibration curve constructed *una tantum* with known concentrations of the Grp94-IgG complex formed *in vitro.* The concentration read from the standard curve must then be corrected for the dilution factor.

### DESCRIPTION OF THE FIGURES

Figure 1. Scheme of the sandwich ELISA in which the capture (primary) antibody is attached to the bottom of the plate and, after the addition of the antigen protein contained in the biological sample, the immune reaction is detected by adding the detection (secondary) antibody directly or indirectly conjugated with a detection system. Ab, antibody; HRP, horseradish peroxidase. A) Sandwich ELISA developed by firms selling ELISA kits for the measurement of Grp94 for the research use only. Both the detection and the capture antibodies are anti-Grp94 antibodies. B) ELISA method matter of the present patent in which the capture antibody is an anti-Grp94 antibody and differs from the detection antibody that recognizes and binds IgG of the Grp94-IgG complex. The presence of the complex is proved by positivity of the immune reaction.
Figure 2. The figure shows the Western blot analysis for Grp94 (Fig. 2a) and IgG (Fig. 2b) made on plasma of cancer patients analyzed in the present investigation. Electrophoresis has been performed in denaturing conditions (SDS-PAGE) by loading on to an 8.5% polyacrylamide gel 10 µg proteins of each plasma sample in nonreducing conditions. Before loading, the protein concentration was determined on dialyzed plasma using a spectrophotometric method. Proteins were then blotted on to a PVDF membrane (Immobilon P, Millipore, Bellerica, MA, USA). After blocking the membrane with 10% milk, the membrane was treated with anti-Grp94 rabbit polyclonal antibodies (Enzo Life Sciences) and then, after stripping of the membrane, with anti-human IgG sheep polyclonal antibodies (Bethyl Laboratories, Inc., Montgomery, TX, USA). Overlapping results were obtained when anti-Grp94 rat monoclonal antibodies (clone 9G10) (Santa Cruz Biotechnologies, Santa Cruz, CA, USA) were used in place of polyclonal antibodies. In the figure only the part of the membrane containing the bands positive for both Grp94 and IgG is shown. Bands focused exclusively at high molecular weights to demonstrate the formation of big complexes.
Figure 3. Measurements of Grp94-IgG complexes made with the ELISA method on plasma of both a patient (Fig. 2, # 11) and a healthy control subject. The figure shows the curves of absorbance, measured at 450 nm, of Grp94-IgG complexes in plasma tested at scalar dilutions (1:512, 1:256, 1:128, 1:64, 1:32, 1:16). The plate was covered with anti-Grp94 polyclonal antibodies (2 µg/ml per well), and plasma samples were then added, in duplicate for each dilution, following the procedure described above, in the text. At variance with the plasma of control subject, whose curve of absorbance shows little or no increase by increasing the plasma concentration, with the patient's plasma a significant increase in the absorbance is observed already at higher dilutions of plasma. The reaction shows linearity up to the dilution of 1:128, whereas at lower dilutions linearity of the reaction is lost due to the saturation of the process. O.D., optical density in arbitrary units (a.u.).
Figure 4. Measurements of Grp94-IgG complexes made with the ELISA method on plasma of 6 patients. Plasma was tested at dilutions of 1:128, 1:32 and 1:16, following the procedure described above, in the text. The coating was the same as that reported in the legend to Fig. 3, and any plasma sample was analyzed in duplicate at each dilution. In the experiment, the plasma of the reference patient (upper curve) already tested at other dilutions (see Fig. 3) was also included. O.D., optical density in arbitrary units (a.u.).
Figure 5. Measurements of Grp94-IgG complexes made with the ELISA method on plasma of 22 patients (13 males and 9 females) with tumor of the gastro-enteric tract, and in 15 healthy control subjects. Each plasma sample was analyzed in duplicate at the fixed dilution of 1:128, following the procedure described above, in the text, and the absorbance measured at 450 nm (O.D., optical density in arbitrary units, a.u.). Horizontal lines in the group of both patients and control subjects indicate the mean of the absorbance values with the mean standard error (numbers of values are reported in the text). The median is 0.34 units of absorbance in the group of control subjects and 0.755 in the group of patients. The 95% confidence interval for control subjects includes values from 0.318 to 0.375, whereas for patients includes values from 0.627 to 0.966. p<0.0001, α level of probability for differences between the means (Mann-Whitney test).
Figure 6. Measurements of Grp94-IgG complexes made with the ELISA method on plasma of 22 patients (as in Fig. 5) after patients were grouped by type of tumor. Horizontal lines in each group represent the mean and the mean standard error: 0.758 (± 0.061) for tumors of the oesophagus and gastro-oesophageal junction (g-e); 0.901 (± 0.284) for gastric tumors; 0.772 (± 0.098) for colon tumors; 0.746 (± 0.069) for sigma/rectum tumors.

## Claims

1. Method of immune-detection of the measurement of endogenous complexes of Glucose-regulated protein94 (Grp94) with Immunoglobulin G (IgG), wherein endogenous complexes of Grp94-IgG are a diagnostic and prognostic tumor marker, and the method for measuring the complexes is a sandwich ELISA that employs a capture or primary antibody, preferentially attached to wells of a plate, able to recognize one or more epitopes of Grp94 of the complex present in the biological sample, and a detection or secondary antibody able to recognize one or more epitopes of IgG of the complex and associated with a detection system employing either an enzyme or substances of another nature.

2. Method of immune-detection of the measurement of endogenous complexes of Glucose-regulated protein94 (Grp94) with Immunoglobulin G (IgG), according to claim 1, wherein the capture or primary antibody and the detection or secondary antibody can be either monoclonal or polyclonal antibodies, either whole antibodies or fragments of them.

3. Method of immune-detection of the measurement of endogenous complexes of Glucose-regulated protein94 (Grp94) with Immunoglobulin G (IgG), according to claims 1 and 2, wherein the detection system preferentially but not exclusively includes: a) an enzyme either directly conjugated with the detection antibody or conjugated with a system of amplification; b) a specific substrate that is degraded by the enzyme into a product of degradation that can be measured by means of a spectrophotometric analysis at a particular wavelength.

## Patentansprüche

1. Verfahren zur Immunerkennung der Messung endogener Komplexe aus glukosereguliertem Protein94 (Grp94) mit Immunoglobulin G (IgG), wobei endogene Komplexe aus Grp94-IgG ein diagnostischer und prognostischer Tumormarker sind, und das Verfahren zur Messung der Komplexe ein ELISA-Sandwich ist, das einen vorzugsweise in Wells einer Platte fixierten Fänger- oder primären Antikörper nutzt, welcher in der Lage ist, ein oder mehrere Grp94-Epitope des in der biologischen Probe vorhandenen Komplexes zu erkennen, und einen Erkennungs- oder sekundären Antikörper, der in der Lage ist, ein oder mehrere IgG-Epitope des Komplexes zu erkennen, und der mit einem Detektionssystem assoziiert ist, das entweder ein Enzym oder Stoffe anderer Art verwendet.

2. Verfahren zur Immunerkennung der Messung endogener Komplexe aus glukosereguliertem Protein94 (Grp94) mit Immunoglobulin G (IgG) nach Patentanspruch 1, wobei der Fänger- oder primäre Antikörper und der Erkennungs- oder sekundäre Antikörper monoklonale oder polyklonale Antikörper sein können, sowohl ganze Antikörper als auch Fragmente derselben.

3. Verfahren zur Immunerkennung der Messung endogener Komplexe aus glukosereguliertem Protein94 (Grp94) mit Immunoglobulin G (IgG) nach den Patentansprüchen 1 und 2, wobei das Detektionssystem vorzugsweise, aber nicht ausschließlich Folgendes umfasst: a) ein entweder direkt mit dem Detektionsantikörper oder mit einem Amplifikationssystem konjugiertes Enzym; b) ein spezifisches Substrat, das durch das Enzym in ein Degradationsprodukt degradiert wird, welches durch eine spektrophotometrische Analyse mit einer bestimmten Wellenlänge gemessen werden kann.

## Revendications

1. Méthode de détection immunitaire de la mesure de complexes endogènes de la protéine régulée par le Glucose 94 (Grp94) avec Immunoglobuline G (IgG), où les complexes endogènes de Grp94-IgG sont un marqueur tumoral diagnostique et pronostique, et la méthode pour la mesure des complexes est une méthode ELISA sandwich qui emploie un anticorps de capture ou primaire, préférablement relié à des puits d'une plaque, en mesure de reconnaître un ou plusieurs épitopes de Grp94 du complexe présent dans l'échantillon biologique, et un anticorps de détection ou secondaire en mesure de reconnaître un ou plusieurs épitopes de IgG du complexe et associé à un système de détection utilisant une enzyme ou des substances de nature différente.

2. Méthode de détection immunitaire de la mesure de complexes endogènes de la protéine régulée par le Glucose 94 (Grp94) avec Immunoglobuline G (IgG), selon la revendication 1, où l'anticorps de capture ou primaire et l'anticorps de détection ou secondaire peuvent être soit des anticorps monoclonaux ou polyclonaux, soit des anticorps entiers ou des fragments de ceux-ci.

3. Méthode de détection immunitaire de la mesure de complexes endogènes de la protéine régulée par le Glucose 94 (Grp94) avec Immunoglobuline G (IgG), selon les revendications 1 et 2, où le système de détection comprend préférablement, mais pas exclusivement : a) une enzyme conjuguée directement avec l'anticorps de détection ou conjuguée à un système d'amplification ; b) un substrat spécifique qui est dégradé par l'enzyme en un produit de dégradation qui peut être mesuré par une analyse spectrophotométrique à une longueur d'onde particulière.
